# EUROPÄISCHE PATENTSCHRIFT

(11) **EP 1 319 389 B1**
(45) Veröffentlichungstag und Bekanntmachung des Hinweises auf die Patenterteilung: **21.03.2007**
(21) Anmeldenummer: 02026710.0
(22) Anmeldetag: 30.11.2002
(51) Int. Cl.: A61K 8/81

(54) **Haarfestiger mit anionischem und/oder amphoterem Filmpolymer**
Hair fixative composition with anionic and/or amphoteric film-forming polymer
Composition capillaire fixante avec un polymère filmogène anionique et/ou amphotère

(30) Priorität: 12.12.2001 DE 10160992
(43) Veröffentlichungstag der Anmeldung: 18.06.2003
(73) Patentinhaber: Beiersdorf AG, 20245 Hamburg (DE)
(72) Erfinder: Primmel, Bettina, 20146 Hamburg (DE); Liebelt, Kerstin, 22529 Hamburg (DE)

(56) Entgegenhaltungen:
- DE-A- 4 239 499
- DE-A- 4 316 242
- DE-A- 19 937 434
- DE-C- 19 957 947

## Beschreibung

Die vorliegende Erfindung betrifft Haarfestiger enthaltend mindestens ein Terpolymer aus Vinylpyrrolidon, Dimethylaminopropylmethacrylamid und quaternisiertem Alkyldimethylaminopropylmethacrylamid in einer Konzentration von 0,1 bis 10 Gewichts-%, mindestens einem anionischen und/oder amphoteren Polymer in einer Konzentration von 0,1 bis 10 Gewichts-%, jeweils bezogen auf das Gesamtgewicht der Zubereitung und deren Verwendung.

Der ganze menschliche Körper mit Ausnahme der Lippen, der Handinnenflächen und der Fußsohlen ist behaart, zum Großteil allerdings mit kaum sichtbaren Wollhärchen. Wegen der vielen Nervenenden an der Haarwurzel reagieren Haare empfindlich auf äußere Einflüsse wie Wind oder Berührung und sind daher ein nicht zu unterschätzender Bestandteil des Tastsinns. Die wichtigste Funktion des menschlichen Kopfhaares dürfte allerdings heute darin bestehen, das Aussehen des Menschen in charakteristischer Weise mitzugestalten. Ähnlich wie die Haut erfüllt es eine soziale Funktion, da es über sein Erscheinungsbild erheblich zum Selbstwertgefühl des Individuums beiträgt.

Das Haar besteht aus dem frei aus der Haut herausragenden Haarschaft - dem keratinisierten (toten) Teil, der das eigentlich sichtbare Haar darstellt - und der in der Haut steckenden Haarwurzel - dem lebenden Teil, in dem das sichtbare Haar ständig neu gebildet wird. Der Haarschaft seinerseits ist aus drei Schichten aufgebaut: einem zentralen Teil - dem sogenannten Haarmark (Medulla), welches allerdings beim Menschen zurückgebildet ist und oft gänzlich fehlt - ferner dem Mark (Cortex) und der äußeren, bis zu zehn Lagen starken Schuppenschicht (Cuticula), die das ganze Haar umhüllt.

Natürliches Haar hängt meist schlaff und ohne Volumen vom Kopf herab. Ein Ziel der Haarpflege ist es daher, dem Haar Fülle und Frisur zu geben. Zur Gestaltung (engl. Styling) vielseitiger Frisuren verwendet man Haarfestiger, meist in Form von Schaumfestigern oder Haarsprays. Beide haarkosmetischen Mittel sind in Ihrer Zusammensetzung ähnlich unterscheiden sich aber in ihrer Aufgabe und Anwendung. Schaumfestiger (auch Haarschaumfestiger genannt) werden in der Regel zur Frisurgestaltung im feuchten Haar verteilt, Haarsprays zur Fixierung auf die (trockene) fertig gestylte Frisur gesprüht. Neben den wegen ihrer leichten und angenehmen Anwendbarkeit zunehmend beliebten Schaumhaarfestigern und flüssigen Haarfestigern werden auch Haarfestigergele angeboten.

Die beiden Grundbausteine von Haarfestigern sind die Filmbildner und das Lösungsmittel. Als Filmbildner werden meist Mischpolymerisate aus Vinylpyrrolidon und Vinylacetat oder neutralisierter Crotonsäure eingesetzt. Filmbildner bleiben nach dem Verdunsten des Lösungsmittels (meist Wasser und/oder Ethanol) als Film auf dem Haar haften und fixieren dessen Form. Haarschaumfestiger enthalten zusätzlich Treibmittel wie Propan, n-Butan und/oder Isobutan.

Je nach gewünschter Festigkeit der Frisur werden unterschiedlich stark festigende Filmbildner in unterschiedlichen Mengenanteilen eingesetzt. Man unterscheidet daher Haarfestiger mit schwacher, normaler und starker Festigung sowie Festiger für normales, trockenes und fettiges Haar [W. Umbach (Hrsg.): Kosmetik, Entwicklung, Herstellung und Anwendung kosmetischer Mittel, 2. Aufl., Thieme Verlag, Stuttgart, 1995].

Herkömmliche Haarfestiger auf Basis von anionischen und/oder amphoteren Polymeren in Kombination mit kationischen Polymeren haben den Nachteil, dass sie den Anforderungen bezüglich ihrer Filmeigenschaften für die Frisur nur unzureichend gerecht werden, wenn gleichzeitig die Kämmbarkeit und der "Griff" des nassen und trockenen Haares den Verbraucherwünschen entsprechen soll. Insbesondere das Entstehen von sogenannten Filmplaken, das heißt Rückständen, die beim Kämmen auf Schulter und Kleidung rieseln und den Anwender ungepflegt erscheinen lassen, läßt sich mit Produkten des Standes der Technik nur unzureichend unterdrücken.

Es war daher die Aufgabe der vorliegenden Erfindung, den Nachteilen des Standes der Technik abzuhelfen und Haarfestigungszubereitungen zu entwickeln, die dem Haar eine hohe Festigkeit sowie einen ausgeprägten Langzeithalt geben, es pflegen und dabei das Auftreten von Filmplaken unterdrücken.

Überraschend wird die Aufgabe gelöst durch Haarfestiger enthaltend
a) mindestens ein Terpolymer aus Vinylpyrrolidon, Dimethylaminopropylmethacrylamid und quaternisiertem Alkyldimethylaminopropylmethacrylamid in einer Konzentration von 0,1 bis 10 Gewichts-%
b) mindestens ein anionisches und/oder amphoteres Polymer in einer Konzentration von 0,1 bis 10 Gewichts-%,
jeweils bezogen auf das Gesamtgewicht der Zubereitung.

Durch erfindungsgemäßen Haarfestiger bekommt das Haar eine sehr starke Festigung bei gleichzeitiger Reduzierung der Rückstandsbildung, d.h. des Auftretens von Filmplaken. Insgesamt erhält der Festigungsfilm auf dem Haar deutlich verbesserte sensorische Filmeigenschaften.

Zwar sind Terpolymere aus Vinylpyrrolidon, Dimethylaminopropylmethacrylamid und quaternisiertem Alkyldimethylaminopropylmethacrylamid und ihre Verwendung in haarfestigenden Mitteln, insbesondere in Aerosol- und Non-Aerosol-Festigungsprodukten in der WO 00 68 282 beschrieben. Die in dieser Schrift offenbarten Zubereitungen zeichnen sich zwar durch gute Filmeigenschaften wie geringe Klebrigkeit und hohe Feuchtigkeitsresistenz auf, ihre Festigungseigenschaften, insbesondere der Langzeithalt läßt jedoch zu wünschen übrig.

Auch die DE 199 57 947 offenbart Terpolymere für haarkosmetische Zubereitungen. Diese Schrift konnte jedoch nicht den Weg zur vorliegenden Erfindung weisen.

Auch anionische und amphotere Polymere sind in Haarfestigungsprodukten an sich bekannt. Meist werden Acrylat-oder Methacrylatderivate eingesetzt. Sie geben dem Haar eine hohe Festigkeit und einen guten Langzeithalt. Nachteilig am Stande der Technik sind jedoch die taktilen Eigenschaften dieser Produkte und das Auftreten von Filmplaken beim Kämmen der Haare.

Zur Untersuchung der Reduzierung von Filmplaken wurden anwendungstechnische Halbseitenuntersuchungen durchgeführt. Die Rückstände wurden gesammelt und ausgezählt. Hierbei ergab die Verwendung von erfindungsgemäßen Haarfestigern eine signifikante Reduzierung der Filmplaken.

Erfindungsgemäß besonders vorteilhaft ist die Konzentration eines Terpolymers aus Vinylpyrrolidon, Dimethylaminopropylmethacrylamid und quarternisiertem Dimethylaminopropylmethacrylamid von 0,1 bis 10 Gewichts-% und insbesondere von 0,25 bis 2,5 Gewichts-%, jeweils bezogen auf das Gesamtgewicht der Zubereitung.

Die erfindungsgemäß vorteilhaften Terpolymere bestehen aus Vinylpyrrolidon, Dimethylaminopropylmethacrylamid und Alkyldimethylaminopropylmethacrylamidoammoniumsalzen.

Alle in der WO 00 68 282 offenbarten Terpolymere sind erfindungsgemäß vorteilhaft.

Ganz besonders vorteilhaft ist dabei das Terpolymer aus Vinylpyrrolidon, Dimethylaminopropylmethacrylamid und Lauryldimethylaminopropylmethacrylamidoammoniumchlorid (z.B. Styleze W-20 der Firma ISP).

Erfindungsgemäß besonders vorteilhaft ist die Konzentration mindestens eines anionischen und/oder amphoteren Polymers von 0,1 bis 10 Gewichts-% und insbesondere von 1 bis 5 Gewichts-%, jeweils bezogen auf das Gesamtgewicht der Zubereitung.

Es ist erfindungsgemäß vorteilhaft, das anionische Polymer aus der Gruppe Vinylacetat/Crotonsäure-Copolymer, Vinylacetat/Acrylat-Copolymer, Vinylacetat/ Vinylneodecanoat/Crotonsäure-Copolymer, Natriumacrylat/Vinylalkohol-Copoly-mer, Natriumpolystyrolsulfat, Ethylacrylat/N-tert.-Butylacrylamid/Acrylsäure-Copolymer, Vinylpyrrolidon/Vinylacetat/Itaconsäure-Copolymer, Acrylsäure/Acryl-amid-Copolymer und deren Natriumsalze, sowie Homo-und/oder Copolymere von Acrylsäure und/oder Methacrylsäure und/oder deren Salze, Acrylat/Hydroxyacrylat-Copolymer, Octylacrylamid/Acrylat-Copolymer, Octylacrylamid/Methacrylsäureester-Copolymer, Butylacrylat/N-Vinylpyrroli-don-Copolymer, Methylvinylether/ Maleinsäure-Copolymer und deren Ethyl-, Isopropyl- und Butylester, Silikon/Acrylsäure oder Methacrylsäure Copolymer, Polyurethane auf Basis von Diisocyanaten mit endständigen Säuregruppen zu wählen.

Erfindungsgemäß besonders vorteilhafte anionische Polymere sind Copolymere aus Ethylacrylat und Methacrylsäure.

Es ist ferner erfindungsgemäß vorteilhaft, das amphotere Polymer aus der Gruppe N-Octylacrylamid/Acrylsäure/tert.-Butylaminoethylmethacrylat-Copolymer, Gruppe N-Octylacrylamid/Methacrylsäure/tert.-Butylaminoethylmethacrylat-Copolymer sowie Copolymere aus Methacryloylbetain / Alkylmethacrylaten, Copolymere aus Monomeren mit Carboxy- und/oder Sulfongruppen, insbesondere Acrylsäure, Methacrylsäure, Itaconsäure und Monomeren mit Aminogruppen, insbesondere Monoalkylaminoalkylacrylate, Dialkylaminoalkylacrylate, Monoalkylaminoalkylmethacrylate, Dialkylaminoalkylmethacrylate, Monoalkylaminoalkylacrylamide, Dialkylaminoalkylacrylamide, Monoalkylaminoalkylmethacrylamide, Dialkylaminoalkylmethacrylamide, und Copolymeren aus N-Octylacrylamid, Methylmethacrylat, Hydroxypropylmethacrylat, N-tert.-Butylaminoethylmethacrylat und Acrylsäure zu wählen.

Die erfindungsgemäß besonders vorteilhaften amphoteren Polymere sind N-Octylacrylamid/Acrylsäure/tert.-Butylaminoethylmethacrylat-Copolymere.

Eine besonders bevorzugte Ausführungsform der erfindungsgemäßen Haarfestiger ist ihr Vorliegen in Form einer wässrig und/oder alkoholischen Lösung. Es ist daher erfindungsgemäß von Vorteil, wenn gleich nicht zwingend, die Säurefunktionen der anionischen und/oder amphoteren Polymere ganz oder teilweise mit einer Base zu neutralisieren. Dies ist insbesondere dann vorteilhaft, wenn die Wasserlöslichkeit beziehungsweise Wasserdispergierbarkeit der Polymere erhöht werden soll. Als Base zur Neutralisation der Säurefunktionen der Polymere dienen erfindungsgemäß basisch reagierende Salze und/oder Hydroxyde von Alkali- und/oder Erdalkalimetallen und/oder Ammoniak, primäre, sekundäre und/oder tertiäre Amine (z.B. Triethanolamin, Triisopropanolamin, Aminomethylpropanol, Aminomethylpropandiol).

Erfindungsgemäß besonders vorteilhaft ist hierbei die Verwendung von Aminomethylpropanol als Base.

Die erfindungsgemäßen Haarfestiger können vorteilhaft mit einem Treibgas aufgeschäumt werden. Dabei ist es vorteilhaft, das Treibgas in einer Menge von 0,5 bis 20 Gewichts-% und besonders bevorzugt in einer Konzenrtation von 5 bis 15 Gewichts-% bezogen auf das Gesamtgewicht der Formulierung einzusetzen.

Die erfindungsgemäß bevorzugten Treibgase sind Propan, Isobutan und n-Butan sowie deren Mischungen. Aber auch Druckluft, Kohlendioxid, Stickstoff, Stickstoffdioxid und Dimethylether sowie Mischungen all dieser Gase sind erfindungsgemäß vorteilhaft zu verwenden.

Natürlich weiß der Fachmann, dass es an sich nichttoxische Treibgase gibt, die grundsätzlich für die Verwirklichung der vorliegenden Erfindung in Form von Aerosolpräparaten geeignet wären, auf die aber dennoch wegen bedenklicher Wirkung auf die Umwelt oder sonstiger Begleitumstände verzichtet werden sollte, insbesondere Fluorkohlenwasserstoffe und Fluorchlorkohlenwasserstoffe (FCKW).

Die kosmetischen und dermatologischen Zubereitungen können erfindungsgemäß kosmetische Hilfsstoffe enthalten, wie sie üblicherweise in solchen Zubereitungen verwendet werden, z.B. Konservierungsmittel, Antioxidanien, Bakterizide, Parfüme, Emulgatoren, weichmachende, anfeuchtende und/oder feuchthaltende Substanzen, Fette, Öle, Wachse, Farbstoffe, Pigmente, die eine färbende Wirkung haben, Verdickungsmittel oder andere übliche Bestandteile einer kosmetischen oder dermatologischen Formulierung wie Alkohole, Polyole, Polymere, Schaumstabilisatoren, Elektrolyte, organische Lösungsmittel oder Silikonderivate. Auch die Einarbeitung von UV-Lichtschutzfiltern ist erfindungsgemäß vorteilhaft. Die aufgeführte Liste an Zusatzstoffen soll selbstverständlich nicht limitierend sein.

Die erfindungsgemäßen Haarfestiger können vorteilhafter Weise in einer Pumpspray- oder Aerosolverpackung vorliegen, welche als Schaumspender dienen und aus denen heraus die Haarfestiger angewendet werden können.

Dem entsprechend sind Schaumspender auf Pumpspay- oder Aerosolverpackungsbasis, welche erfindungsgemäße Haarfestiger enthalten ebenso Bestandteil der Erfindung.

Aber auch die Aufbewahrung in Quetschflaschen und anderen in der Haarpflege eingesetzten Verpackungsbehältnissen in denen die erfindungsgemäßen Haarfestiger in Form von Lösungen oder Emulsionen vorliegen und aus diesen heraus angewendet werden können, ist erfindungsgemäß vorteilhaft.

Außerdem ist erfindungsgemäß die Verwendung der erfindungsgemäßen Haarfestiger als Schaumfestiger, Flüssigfestiger, Stylinggel oder Schaumaerosol.

Die Verwendung anionischer und/oder amphoterer Polymerer zur Verbesserung des Langzeithaltes und der Griffeigenschaften des Haares sowie zur Reduzierung der Entstehung von Filmplaken nach der Anwendung von kosmetischen und/oder dermatologischen Zubereitungen für das Haar enthaltend Terpolymer aus Vinylpyrrolidon, Dimethylaminopropylmethacrylamid und quarternisiertem Dimethylaminopropylmethacrylamid ist ebenso erfindungsgemäß wie die Verwendung von Terpolymer aus Vinylpyrrolidon, Dimethylaminopropyl-methacrylamid und quarternisiertem Dimethylaminopropylmethacrylamid zur Verbesserung des Langzeithaltes und der Griffeigenschaften des Haars sowie zur Reduzierung der Entstehung von Filmplaken nach der Anwendung kosmetischer und/oder dermatologischer Zubereitungen für das Haar enthaltend anionische und/oder amphotere Polymere.

Zur Untersuchung der Griffeigenschaften wurden anwendungstechnische Halbseitenuntersuchungen durchgeführt. Einen verbesserten Griff konnte durch den Einsatz von erfindungsgemäßen Zubereitungen nachgewiesen werden.

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

**Schaumfestiger der Festigungsstärke ultra starker Halt**

| | **1** | **2** | **3** | **4** |
|---|---|---|---|---|
| Vinylpyrrolidon, Dimethylaminopropylmethacrylamid, Lauryldimethylaminopropylmethacrylamidoammoniumchlorid, (Styleze W-20 der Firma ISP) | 1 | 1 | 0,5 | 0,5 |
| Ethylacrylat/Methacrylsäure Copolymer Luviflex Soft, (BASF) | 3 | | 4 | |
| N-Octylacrylamid/Acrylsäure/tert.-Butylaminoethylmethacrylat-Copolymer, Amphomer, (National Starch) | | 3 | | 4 |
| Aminomethylpropanol | 0,5 | 0,5 | 0,5 | 0,5 |
| Cetyltrimethylammoniumchlorid | 0,3 | 0,3 | 0,5 | 0,5 |
| Parfüm, Lösungsvermittler, pH-Einstellung, Konservierung | q.s. | q.s. | q.s. | q.s. |
| Propan/Butan | 10 | 10 | 10 | 10 |
| Ethanol | | | 15 | 15 |
| Wasser | ad 100 | ad 100 | ad 100 | ad 100 |

**Styling-Gele der Festigungsstärke ultra starker Halt**

| | **1** | **2** | **3** | **4** |
|---|---|---|---|---|
| Vinylpyrrolidon, Dimethylaminopropylmethacrylamid, Lauryldimethylaminopropylmethacrylamidoammoniumchlorid, (Styleze W-20 der Firma ISP) | 1 | 1 | 0,5 | 0,5 |
| Ethylacrylat/Methacrylsäure Copolymer Luviflex Soft, (BASF) | 3,5 | | 4 | |
| N-Octylacrylamid/Acrylsäure/tert.-Butylaminoethylmethacrylat-Copolymer, Amphomer, (National Starch) | | 3,5 | | 4 |
| Aminomethylpropanol | 0,5 | 0,5 | 0,5 | 0,5 |
| Hydroxyethylcellulose | 0,35 | 0,35 | 0,35 | 0,35 |
| Propylenglykol | 0,5 | 0,5 | 0,5 | 0,5 |
| Cetyltrimethylammoniumchlorid | 0,3 | 0,3 | 0,1 | 0,1 |
| Parfüm, Lösungsvermittler, pH-Einstellung, Konservierung | q.s. | q.s. | q.s. | q.s. |
| Propan/Butan | 10 | 10 | 10 | 10 |
| Ethanol | | | 15 | 15 |
| Wasser | ad 100 | ad 100 | ad 100 | ad 100 |

## Patentansprüche

1. Haarfestiger enthaltend
a) mindestens ein Terpolymer aus Vinylpyrrolidon, Dimethylaminopropylmethacrylamid und quaternisiertem Alkyldimethylaminopropylmethacrylamid in einer Konzentration von 0,1 bis 10 Gewichts-%
b) mindestens ein anionisches und/oder amphoteres Polymer in einer Konzentration von 0,1 bis 10 Gewichts-%,
jeweils bezogen auf das Gesamtgewicht der Zubereitung.

2. Haarfestiger nach Anspruch 1, **dadurch gekennzeichnet, dass** das anionische Polymer gewählt wird aus der Gruppe Vinylacetat/Crotonsäure-Copolymer, Vinylacetat/Acrylat-Copolymer, Vinylacetat/Vinylneodecanoat/Crotonsäure-Copolymer, Natriumacrylat/Vinylalkohol-Copolymer, Natriumpolystyrolsulfat, Ethylacrylat/N-tert.-Butylacrylamid/Acrylsäure-Copolymer, Vinylpyrrolidon/Vinylacetat/ltaconsäure-Copolymer, Acrylsäure/Acrylamid-Copolymer und deren Natriumsalze, sowie Homo-und Copolymere von Acrylsäure und/oder Methacrylsäure und/oder deren Salze, Acrylat/Hydroxyacrylat-Copolymer, Octylacrylamid/Acrylat-Copolymer, Octylacrylamid/Methacrylsäureester-Copolymer, Butylacrylat/N-Vinylpyrrolidon-Copolymer, Methylvinylether/Maleinsäure-Copolymer und deren Ethyl-, Isopropyl- und Butylester, Silikon/Acrylsäure oder Methacrylsäure Copolymer, Diglcol/Cyclohexandimethanol/lsophthalat/Sulfoisophthalat Copolymer, Polyurethane auf Basis von Diisocyanaten mit endständigen Säuregruppen.

3. Haarfestiger nach einem der Ansprüche 1 oder 2, **dadurch gekennzeichnet, dass** das amphotere Polymer gewählt wird aus der Gruppe N-Octylacrylamid/Acrylsäure/tert.-Butylaminoethylmethacrylat-Copolymer, Gruppe N-Octylacrylamid/Methacrylsäure/tert.-Butylaminoethylmethacrylat-Copolymer sowie Copolymere aus Methacryloylbetain, Alkylmethacrylaten, Monomeren mit Carboxy- und/oder Sulfongruppen, insbesondere Acrylsäure, Methacrylsäure, Itaconsäure, Monomere mit Aminogruppen, insbesondere Monoalkylaminoalkylacrylate, Dialkylaminoalkylacrylate, Monoalkylaminoalkylmethacrylate, Dialkylaminoalkylmethacrylate, Monoalkylaminoalkylacrylamide, Dialkylaminoalkylacrylamide, Monoalkylaminoalkylmethacrylamide, Dialkylaminoalkylmethacrylamide, und Copolymeren aus N-Octylacrylamid, Methylmethacrylat, Hydroxypropylmethacrylat, N-tert.-Butylaminoethylmethacrylat und Acrylsäure.

4. Haarfestiger nach einem der Ansprüche 1 bis 3, **dadurch gekennzeichnet, dass** die Säurefunktionen der anionischen und/oder amphoteren Polymere ganz oder teilweise mit einer Base neutralisiert sind.

5. Haarfestiger nach Anspruch 4 **dadurch gekennzeichnet dass** als Base zur Neutralisation der Säurefunktionen der Polymere basisch reagierende Salze und/oder Hydroxyde von Alkali- und/oder Erdalkalimetallen und/oder Ammoniak, primäre, sekundäre und/oder tertiäre Amine eingesetzt werden.

6. Haarfestiger nach einem der Ansprüche 1 bis 5, **dadurch gekennzeichnet, dass** die Zubereitung mit einem Treibgas aufgeschäumt wird.

7. Haarfestiger nach einem der Ansprüche 1 bis 6, **dadurch gekennzeichnet, dass** das Treibgas in einer Menge von 0,5 bis 20 Gewichts-% bezogen auf das Gesamtgewicht der Formulierung eingesetzt wird.

8. Haarfestiger nach einem der Ansprüche 1 bis 7, **dadurch gekennzeichnet, dass** als Treibgas Propan, Isobutan und/oder n-Butan eingesetzt wird.

9. Haarfestiger nach einem der Ansprüche 1 bis 8, **dadurch gekennzeichnet, dass** die Zubereitung weitere Wirk-, Hilfs und/oder Zusatzstoffe enthält.

10. Haarfestiger nach einem der Ansprüche 1 bis 9, **dadurch gekennzeichnet, dass** die Zubereitung in einer Pumpspray- oder Aerosolverpackung als Schaumspender vorliegt, aus der heraus sie angewendet wird.

11. Schaumspender auf Pumpspay- oder Aerosolverpackungsbasis, enthaltend einen Haarfestiger nach einem der Ansprüche 1 bis 10.

12. Verwendung eines Haarfestigers nach einem der vorhergehenden Ansprüche als Schaumfestiger, Flüssigfestiger, Stylinggel oder Schaumaerosol.

13. Verwendung anionischer und/oder amphoterer Polymere nach Anspruch 1 bis 3 zur Verbesserung des Langzeithaltes, der Griffeigenschaften und der sensorischen Filmeigenschaften des Haars sowie zur Reduzierung der Entstehung von Filmplaken nach der Anwendung von kosmetischen und/oder dermatologischen Zubereitungen für das Haar enthaltend Terpolymer aus Vinylpyrrolidon, Dimethylaminopropylmethacrylamid und quaternisiertem Dimethylaminopropylmethacrylamid.

14. Verwendung von Terpolymer aus Vinylpyrrolidon, Dimethylaminopropylmethacrylamid und quarternisiertem Dimethylaminopropylmethacrylamid nach Anspruch 1 bis 3 zur Verbesserung des Langzeithaltes, der Griffeigenschaften und der sensorischen Filmeigenschaften des Haars sowie zur Reduzierung der Entstehung von Filmplaken nach der Anwendung kosmetischer und/oder dermatologischer Zubereitungen für das Haar enthaltend anionische und/oder amphotere Polymere.

## Claims

1. Hair fixative composition comprising
a) at least one terpolymer of vinylpyrrolidone, dimethylaminopropylmethacrylamide and quaternized alkyldimethylaminopropylmethacrylamide in a concentration of from 0.1 to 10% by weight
b) at least one anionic and/or amphoteric polymer in a concentration of from 0.1 to 10% by weight,
in each case based on the total weight of the preparation.

2. Hair fixative composition according to Claim 1, **characterized in that** the anionic polymer is chosen from the group vinyl acetate/crotonic acid copolymer, vinyl acetate/acrylate copolymer, vinyl acetate/vinyl neodecanoate/crotonic acid copolymer, sodium acrylate/vinyl alcohol copolymer, sodium polystyrene sulphate, ethyl acrylate/N-tert-butylacrylamide/acrylic acid copolymer, vinylpyrrolidone/vinyl acetate/itaconic acid copolymer, acrylic acid/acrylamide copolymer and sodium salts thereof, and homo- and copolymers of acrylic acid and/or methacrylic acid and/or salts thereof, acrylate/hydroxyacrylate copolymer, octylacrylamide/acrylate copolymer, octylacrylamide/methacrylic ester copolymer, butyl acrylate/N-vinylpyrrolidone copolymer, methyl vinyl ether/maleic acid copolymer and the ethyl, isopropyl and butyl esters thereof, silicone/acrylic acid or methacrylic acid copolymer, diglycol/cyclohexanedimethanol/isophthalate/sulphoisophthalate copolymer, polyurethanes based on diisocyanates with terminal acid groups.

3. Hair fixative composition according to one of Claims 1 or 2, **characterized in that** the amphoteric polymer is chosen from the group N-octylacrylamide/acrylic acid/tert-butylaminoethyl methacrylate copolymer, N-octylacrylamide/methacrylic acid/tert-butylaminoethyl methacrylate copolymer, and copolymers of methacryloylbetaine, alkyl methacrylates, monomers with carboxy and/or sulpho groups, in particular acrylic acid, methacrylic acid, itaconic acid, monomers with amino groups, in particular monoalkylaminoalkyl acrylates, dialkylaminoalkyl acrylates, monoalkylaminoalkyl methacrylates, dialkylaminoalkyl methacrylates, monoalkylaminoalkylacrylamides, dialkylaminoalkylacrylamides, monoalkylaminoalkylmethacrylamides, dialkylaminoalkylmethacrylamides, and copolymers of N-octylacrylamide, methyl methacrylate, hydroxypropyl methacrylate, N-tert-butylaminoethyl methacrylate and acrylic acid.

4. Hair fixative composition according to one of Claims 1 to 3, **characterized in that** the acid functions of the anionic and/or amphoteric polymers are completely or partially neutralized with a base.

5. Hair fixative composition according to Claim 4, **characterized in that** basic salts and/or hydroxides of alkali metals and/or alkaline earth metals and/or ammonia, primary, secondary and/or tertiary amines are used as base for neutralizing the acid functions of the polymers.

6. Hair fixative composition according to one of Claims 1 to 5, **characterized in that** the preparation is foamed with a propellant gas.

7. Hair fixative composition according to one of Claims 1 to 6, **characterized in that** the propellant gas is used in an amount of from 0.5 to 20% by weight, based on the total weight of the formulation.

8. Hair fixative composition according to one of Claims 1 to 7, **characterized in that** the propellant gas used is propane, isobutane and/or n-butane.

9. Hair fixative composition according to one of Claims 1 to 8, **characterized in that** the preparation comprises further active ingredients, auxiliaries and/or additives.

10. Hair fixative composition according to one of Claims 1 to 9, **characterized in that** the preparation is in the form of a pump spray or aerosol packaging as foam dispenser from which it is applied.

11. Foam dispenser on a pump spray or aerosol packaging basis comprising a hair fixative composition according to one of Claims 1 to 10.

12. Use of a hair fixative composition according to one of the preceding claims as foam fixative composition, liquid fixative composition, styling gel or foam aerosol.

13. Use of anionic and/or amphoteric polymers according to Claim 1 to 3 for improving the long-term hold, the feel properties and the sensory film properties of the hair and for reducing the formation of film flakes following the use of cosmetic and/or dermatological preparations for the hair comprising terpolymer of vinylpyrrolidone, dimethylaminopropylmethacrylamide and quaternized dimethylaminopropylmethacrylamide.

14. Use of terpolymer of vinylpyrrolidone, dimethylaminopropylmethacrylamide and quaternized dimethylaminopropylmethacrylamide according to Claim 1 to 3 for improving the long-term hold, the feel properties and the sensory film properties of the hair and for reducing the formation of film flakes following the use of cosmetic and/or dermatological preparations for the hair comprising anionic and/or amphoteric polymers.

## Revendications

1. Composition capillaire fixante comprenant
a) au moins un terpolymère de vinylpyrrolidone, de diméthylaminopropylméthacrylamide et d'un alkyldiméthylaminopropylméthacrylamide quaternisé en une concentration de 0,1 à 10 % en poids,
b) au moins un polymère anionique et/ou amphotère en une concentration de 0,1 à 10 % en poids,
dans chaque cas par rapport au poids total de la préparation.

2. Composition capillaire fixante selon la revendication 1, **caractérisée en ce que** le polymère anionique est choisi parmi le groupe constitué d'un copolymère d'acétate de vinyle/acide crotonique, d'un copolymère d'acétate de vinyle/acrylate, d'un copolymère d'acétate de vinyle/néodécanoate de vinyle/acide crotonique, d'un copolymère d'acrylate de sodium/alcool vinylique, d'un polystyrène-sulfate de sodium, d'un copolymère d'acrylate d'éthyle/N-tert-butylacrylamide/acide acrylique, d'un copolymère de vinylpyrrolidone/acétate de vinyle/acide itaconique, d'un copolymère d'acide acrylique/acrylamide et de ses sels de sodium, ainsi que d'homopolymères et de copolymères d'acide acrylique et/ou d'acide méthacrylique et/ou de leurs sels, d'un copolymère d'acrylate/hydroxyacrylate, d'un copolymère d'octylacrylamide/acrylate, d'un copolymère d'octylacrylamide/ester d'acide méthacrylique, d'un copolymère d'acrylate de butyle/N-vinylpyrrolidone, d'un copolymère d'éther méthylvinylique/acide maléique et de ses esters éthyliques, isopropyliques et butyliques, d'un copolymère de silicone/acide acrylique ou acide méthacrylique, d'un copolymère de diglycol/cyclohexanediméthanol/isophtalate/sulfo-isophtalate, de polyuréthanes à base de diisocyanates renfermant des groupes acides terminaux.

3. Composition capillaire fixante selon l'une quelconque des revendications 1 ou 2, **caractérisée en ce que** le polymère amphotère est choisi parmi le groupe constitué d'un copolymère de N-octylacrylamide/acide acrylique/méthacrylate de tert-butylaminoéthyle, d'un copolymère de N-octylacrylamide/acide méthacrylique/méthacrylate de tert-butylaminoéthyle ainsi que de copolymères de méthacryloylbétaïne, de méthacrylates d'alkyle, de monomères renfermant des groupes carboxy et/ou sulfo, en particulier de l'acide acrylique, de l'acide méthacrylique, de l'acide itaconique, de monomères renfermant des groupes amino, en particulier d'acrylates de monoalkylaminoalkyle, d'acrylates de dialkylaminoalkyle, de méthacrylates de monoalkylaminoalkyle, de méthacrylates de dialkylaminoalkyle, de monoalkylaminoalkylacrylamides, de dialkylaminoalkylacrylamides, de monoalkylaminoalkylméthacrylamides, de dialkylaminoalkylméthacrylamides, et de copolymères de N-octylacrylamide, de méthacrylate de méthyle, de méthacrylate d'hydroxypropyle, de méthacrylate de N-tert-butylaminoéthyle et d'acide acrylique.

4. Composition capillaire fixante selon l'une quelconque des revendications 1 à 3, **caractérisée en ce que** les fonctions acides des polymères anioniques et/ou amphotères sont totalement ou partiellement neutralisées avec une base.

5. Composition capillaire fixante selon la revendication 4, **caractérisée en ce que** l'on utilise, en tant que base pour la neutralisation des fonctions acides des polymères, des sels réagissant de façon basique et/ou des hydroxydes de métaux alcalins et/ou alcalino-terreux et/ou l'ammoniac, des amines primaires, secondaires et/ou tertiaires.

6. Composition capillaire fixante selon l'une quelconque des revendications 1 à 5, **caractérisée en ce que** la préparation est moussée avec un gaz propulseur.

7. Composition capillaire fixante selon l'une quelconque des revendications 1 à 6, **caractérisée en ce que** le gaz propulseur est utilisé en une quantité de 0,5 à 20 % en poids, par rapport au poids total de la formulation.

8. Composition capillaire fixante selon l'une quelconque des revendications 1 à 7, **caractérisée en ce que** l'on utilise, en tant que gaz propulseur, du propane, de l'isobutane et/ou du n-butane.

9. Composition capillaire fixante selon l'une quelconque des revendications 1 à 8, **caractérisée en ce que** la préparation comprend des ingrédients actifs, des auxiliaires et/ou des additifs supplémentaires.

10. Composition capillaire fixante selon l'une quelconque des revendications 1 à 9, **caractérisée en ce que** la préparation se présente sous la forme d'un conditionnement pulvérisateur à pompe ou à aérosol en tant que distributeur de mousse, à partir duquel elle est appliquée.

11. Distributeur de mousse à base d'un conditionnement pulvérisateur à pompe ou à aérosol, comprenant une composition capillaire fixante selon l'une quelconque des revendications 1 à 10.

12. Utilisation d'une composition capillaire fixante selon l'une quelconque des revendications précédentes sous la forme d'une composition fixante à mousse, d'une composition fixante liquide, d'un gel coiffant ou d'un aérosol à mousse.

13. Utilisation de polymères anioniques et/ou amphotères selon les revendications 1 à 3 pour l'amélioration de la tenue à longue terme, des propriétés au toucher et des propriétés sensorielles de film des cheveux ainsi que pour la réduction de la formation de plaques de film après l'application de préparations cosmétiques et/ou dermatologiques pour les cheveux, comprenant un terpolymère de vinylpyrrolidone, de diméthylaminopropylméthacrylamide et de diméthylaminopropylméthacrylamide quaternisé.

14. Utilisation d'un terpolymère de vinylpyrrolidone, de diméthylaminopropylméthacrylamide et de diméthylaminopropylméthacrylamide quaternisé selon les revendications 1 à 3 pour l'amélioration de la tenue à longue terme, des propriétés au toucher et des propriétés sensorielles de film des cheveux ainsi que pour la réduction de la formation de plaques de film après l'application de préparations cosmétiques et/ou dermatologiques pour les cheveux, comprenant des polymères anioniques et/ou amphotères.
